# EUROPÄISCHE PATENTANMELDUNG

(11) **EP 0 603 112 A1**
(43) Veröffentlichungstag der Anmeldung: **22.06.1994**
(21) Anmeldenummer: 93710021.2
(22) Anmeldetag: 04.12.1993
(51) Int. Cl.: A61K 37/64

(54) **Verwendung von Thrombin-Inhibitoren zur Inhibition okularer Fibrinbildung**

(30) Priorität: 17.12.1992 DE 4242655
(71) Anmelder: BEHRINGWERKE Aktiengesellschaft, 35001 Marburg (DE)
(72) Erfinder: Römisch, Jürgen, D-35041 Marburg (DE); Pâques, Eric-Paul, D-35041 Marburg (DE); Schüler, Eckhard, Dr., D-35041 Marburg (DE)

(57) **Zusammenfassung**

Es wird die Verwendung von Substanzen, die die Aktivität loslichen und fibrin- und zellgebundenen Thrombins inhibieren können, im besonderen Hirudin, dessen Derivate, von ihm abgeleitete Peptide und niedermolekulare Thrombininhibitoren in einem Verfahren zur Herstellung eines Arzneimittels zur Inhibition okularer Fibrinbildung und -ablagerung beschrieben.

## Beschreibung

Die Erfindung betrifft die Verwendung von Substanzen, die die Aktivität löslichen und fibrin- und zellgebundenen Thrombins inhibieren können, im besonderen Hirudin, dessen Derivate, von ihm abgeleitete Peptide und niedermolekulare Thrombininhibitoren in einem Verfahren zur Herstellung eines Arzneimittels zur Inhibition okularer Fibrinbildung und -ablagerung.

Die Aktivierung von Fibrinogen zu Fibrin stellt die zentrale Reaktion zur Bildung eines primären Wundverschlusses dar. Dieser so wichtige Vorgang nach Verletzung des Blutgefäßsystemes kann bei unkontrollierter Fibrinbildung und -ablagerung (Thrombus) physiologische Funktionen erheblich beeinträchtigen.

Den entscheidenden Beitrag zur Fibrinogenaktivierung leistet das Thrombin, die zentrale Protease der Blutgerinnung. Fibrinbildung und -ablagerung treten an vaskulären Verletzungsorten auf und setzen sich auch in extravasale Bereiche fort, wenn Plasmakomponenten, darunter Fibrinogen, das Blutgefäßsystem verlassen (zum Beispiel bei erhöhter Permeabilität des Endothels) wie zum Beispiel im Verlauf bestimmter Krankheiten oder bei oder nach operativen Eingriffen. Diese Fibrinablagerungen oder Thromben können nach einiger Zeit durch das körpereigene fibrinolytische System aufgelöst werden. Anders ist es zumeist bei bestimmten Augenkrankheiten oder nach Augenoperationen, in deren Verlauf oder wo post-operativ erhebliche Beeinträchtigungen der Sehkraft durch Fibrin auftreten können (zum Beispiel Trübung, Opaleszenz, "Starburst" Fffekt"), zum Teil verbunden mit Schmerzen. Die körpereigene Fibrinolyse ist intraokular aber nicht oder nur sehr schwach wirksam, wodurch die Beeinträchtigungen häufig sehr lange dauern und zu einer Erblindung führen können.

Therapeutisch kann der Fibrin-/Thrombusabbau durch Applikation von fibrinolytisch wirksamen Substanzen wie Gewebe-Plasminogenaktivator (t-PA) initiiert oder beschleunigt werden. Eine derartige Behandlung kann sich aber nachteilig auf Wundheilungsprozesse auswirken. Bekannt ist zudem, daß während dieser Abbauprozesse freigesetzte Fibrin-Spaltprodukte chemotaktisch auf Makrophagen und Granulozyten wirken und so die Gefahr einer intraokularen Entzündung erheblich erhöhen.

Es ist daher zweckmäßig, prophylaktische Maßnahmen gegen Fibrinbildungen und -ablagerungen zu treffen. Die initiale Aktivierung vonFibrinogen kann durch Hemmung der Aktivität löslichen Thrombins weitgehend unterbunden werden wie in EP-A-0 353 018 für den Plasma-Inhibitor Antithrombin 111 (AT 111) beschrieben. Erst in Kombination mit seinem Kofaktor Heparin entfaltet AT 111 seine volle Wirksamkeit. Die Effektivität dieser Substanzen ist jedoch dadurch limitiert, daß sie nur lösliches Thrombin ausreichend hemmen können. Dagegen werden Thrombinmoleküle, die an Fibrin oder Zelloberflächen gebunden sind, durch AT III/Heparin schlecht gehemmt. Solche oberflächengebundenen Thrombinaktivitäten stellen bekanntermaßen ein erhebliches Problem dar, da sie weitere Fibrinablagerungen verursachen. Zusätzlich aktivieren sie weitere Gerinnungsfaktoren und erhöhen so das Risiko okularer Thromben bei Augenkrankheiten und -operationen.

Es bestand daher die Aufgabe, eine okulare Thromboseprophylaxe mit Substanzen zu schaffen, die neben löslichen auch fibrin- und zellgebundenes Thrombin inhibieren können.

Gegenstand der Erfindung ist die Verwendung von Substanzen, die die Aktivität löslichen und fibrin-und zellgebundenen Thrombins inhibieren können, in einem Verfahren zur Herstellung eines Arzneimittels zur Inhibition okularer Fibrinbildung und -ablagerung.

Bevorzugte solche Substanzen sind Hirudin, dessen Derivate, von ihm abgeleitete Peptide oder niedermolekulare Thrombininhibitoren.

Hirudin ist ein 7 kDa großer, spezifischer Thrombininhibitor aus dem Egel Hirudo medicinalis und kann gentechnisch hergestellt werden, der unabhängig von Kofaktoren sowohl lösliches als auch oberflächenassoziiertes Thrombin effektiv hemmt. Ebenfalls geeignet sind Derivate des Hirudins, beispielsweise solche, die Teilsequenzen aus der Aminosäuresequenz des Hirudins oder Variationen dieser Sequenz enthalten, ein Analogon oder allgemein funktionale Äquivalente des Hirudins oder niedermolekulare Thrombin-Inhibitoren mit den oben beschriebenen Eigenschaften wie das (2R,4R)-4-methyl-1-[N-[(3-methyl-1,2,3,4-tetrahydro-8-quinolinyl)sulfonyl]-L-argi nyl]-2-piperidin und in EP-A-0 097 630 oder 0 468 231 beschriebene Substanzen.

Bei bereits bestehenden okularen Fibrinablagerungen ist die Anwendung dieser Substanzen ebenfalls sinnvoll, da die gegenläufigen Prozesse der Fibrinablagerung und körpereigener Thrombusauflösung zugunsten der Fibrinolyse verschoben werden. Die therapeutische Applikation der entsprechenden Thrombininhibitoren mit fibrinolytisch wirksamen Substanzen beispielsweise intraokular mit t-PAfindet aus den genannten Gründen ebenfalls Verwendung.

Verwendung finden diese Inhibitoren z. B. bei/nach Katarakt, Glaukomoperationen, Vitrektomien und laserchirurgischen Eingriffen (z. B. Photokoagulation, Laser-Ablation), in deren Verlauf häufig Hyphaemen (Augenkammerblutungen) mit folgender Fibrinablagerung/Thrombusbildung auftreten. Dadurch werden u. a. Glaukombildungen (Störungen des Flüssigkeitsablaufes) begünstigt. Auch nach Linsenimplantationen finden die beschriebenen Inhibitoren Anwendung, da die resultierenden Fibrinablagerungen (hervorgerufen u. a. auch durch die künstlichen Oberflächen) eine Iridocyclitis, eine Entzündung der Iris und des Ziliarkörpers, verursachen können. Nicht selten sind auch retinaleArterienbzw. Venenthrombosen (z. B. im Verlauf eines Diabetes mellitus, Autoimmunerkrankungen oder nach Operationen), die durch die Inhibitoren vermieden werden können. In Kombination mit Volumensubstituenten, wie z. B. Hyaluronsäure, können diese Inhibitoren beispielsweise bei chirurgischen Eingriffen Verwendung finden. Zur Prophylaxe oder Therapie entzündlicher oder infektiös bedingter Augenirritationen oder -krankheiten bieten sich entsprechend Kombinationen mit steroidalen oder nichtsteroidalen Antiinflammatorika oder Antibiotika an.

Außerdem können zusammen mit dem Thrombin-Inhibitor Inhibitoren des Plasmins sowie Plasminogen-Aktivator-Inhibitoren (Beispiele: PAI-1 und PAI-2), besonders Inhibitoren der Urokinase (Proteine und niedermolekulare Substanzen) angewandt werden.

Ein Arzneimittel, das diese Thrombin-Inhibitoren und gegebenenfalls weitere der angegebenen Wirkstoffe enthält, kann intravenös, oral, intraokular oder topisch (Augentropfen) vor, bei oder nach Operationen angewandt werden. Es kann einen Thrombin-Inhibitor in einer Menge von 0,01 bis 20 mg/kg Körpergewicht (orale oder i.v. Applikation), bevorzugt 0,05 bis 5 mg/kg Körpergewicht, und von 0,01 bis 100 µg/kg Körpergewicht (intraokular, topisch), bevorzugt 0,1 bis 10 µg/kg Körpergewicht, enthalten.

Die Wirkstoffe, die zusätzlich zu den Thrombin- Inhibitoren angewandt werden, können in einer Menge von 0,001 bis 50 mg/kg (oral / i.v.), bevorzugt 0.01 bis 10 mg/kg, und von 0,001 bis 100 µg/kg, bevorzugt 0.01 bis 10 µg/kg (intraokular, topisch) verwendet werden.

## Patentansprüche

1. Verwendung von Substanzen, die die Aktivität löslichen und fibrin- und zellgebundenen Thrombins inhibieren können, in einem Verfahren zur Herstellung eines Arzneimittels zur Inhibition okularer Fibrinbildung und -ablagerung.

2. Verwendung nach Anspruch 1, dadurch gekennzeichnet, daß der Thrombininhibitor Hirudin, eines seiner Derivate oder ein von ihm abgeleitetes Peptid ist, die Sequenzen aus derAminosäuresequenz des Hirudins enthalten, oder ein Analogon oder allgemein ein funktionales Äquivalent des Hirudins ist.

3. Verwendung nach Anspruch 1, dadurch gekennzeichnet, daß der Thrombininhibitor ein niedermolekularer Thrombininhibitor ist.

4. Verwendung nach Anspruch 1, dadurch gekennzeichnet, daß der Thrombininhibitor in einer Konzentration von 0,01 bis 20 mg/kg Körpergewicht (orale oder i. v. Applikation) odervon 0,01 bis 100 µg/kg Körpergewicht (intraokular, topisch) verwendet wird.

5. Verwendung nach Anspruch 1, dadurch gekennzeichnet, daß der Thrombin-Inhibitor in einer Konzentration von 0,05 bis 5 mg/kg Körpergewicht (orale oder i. v. Applikation) und von 0,1 bis 10 µg/kg Körpergewicht (intraokular, topisch) verwendet wird.

6. Verwendung nach Anspruch 1, dadurch gekennzeichnet, daß ein Arzneimittel oder eine Verpackungseinheit hergestellt wird, das oder die zusätzlich einen Plasmin-Inhibitor oder einen Plasminogen-Aktivator-Inhibitor enthält.

7. Verwendung nach Anspruch 1, dadurch gekennzeichnet, daß ein Arzneimittel oder eine Verpackungseinheit hergestellt wird, das oder die zusätzlich 0,001 bis 50 mg/kg Körpergewicht (oral/i.v.) oder 0,001 bis 100 µg/kg Körpergewicht (intraocular, topisch) eines Plasmin-Inhibitors oder eines Plasminogen-Aktivator-Inhibitors enthält.
